# EUROPÄISCHE PATENTSCHRIFT

(11) **EP 3 125 819 B1**
(45) Veröffentlichungstag und Bekanntmachung des Hinweises auf die Patenterteilung: **03.03.2021**
(21) Anmeldenummer: 15725226.3
(22) Anmeldetag: 30.03.2015
(51) Int. Cl.: A61C 13/00, A61C 5/77

(54) **VERFAHREN ZUR HERSTELLUNG EINER KÜNSTLICHEN ZAHNKRONE, ZAHNKRONE SOWIE ÜBERDECKUNG MIT DIESER ZAHNKRONE**
METHOD FOR PRODUCING AN ARTIFICIAL TOOTH CROWN, TOOTH CROWN AND COVER USING THIS TOOTH CROWN
PROCÉDÉ DE FABRICATION D'UNE COURONNE DENTAIRE PROTHÉTIQUE, COURONNE DENTAIRE ET COUVERTURE DENTAIRE UTILISANT CETTE COURONNE

(30) Priorität: 28.03.2014 DE 102014004435
(43) Veröffentlichungstag der Anmeldung: 08.02.2017
(73) Patentinhaber: VFM Dentallabor GmbH, 22453 Hamburg (DE)
(72) Erfinder: VON FEHRENTHEIL, Alexander, 22527 Hamburg (DE); LOEWE, Johann Philipp, 22761 Hamburg (DE)
(74) Vertreter: Heinemeyer, Karsten
(86) Internationale Anmeldenummer: PCT/EP2015/000674
(87) Internationale Veröffentlichungsnummer: WO 2015/144316

(56) Entgegenhaltungen:
- WO-A1-2008/114142
- WO-A1-2013/127931
- US-A- 5 939 211
- US-A1- 2011 045 436

## Beschreibung

Die Erfindung betrifft ein Verfahren zur Herstellung einer künstlichen Zahnkrone, die einen Grundkörper aus einem biokeramischen Hochleistungskomposit aufweist. Ferner betrifft die Erfindung eine künstliche Zahnkrone sowie eine Überdeckung mit einer entsprechend künstlichen Zahnkrone.

Aus dem Stand der Technik ist eine Vielzahl von künstlichen Zahnkronen sowie Verfahren zu deren Herstellung bekannt. Künstliche Zahnkronen stellen hierbei Abdeckungen dar, die auf Zahnstümpfen, Stiftaufbauten oder auch auf Zahnimplantaten befestigt werden. Für die Herstellung einer künstlichen Zahnkrone wird üblicherweise zunächst der präparierte Zahnstumpf des kranken Zahnes mithilfe einer speziellen Abformmasse abgeformt. Auf der Grundlage dieser Abformung wird dann durch einen Zahntechniker ein Arbeitsmodell erstellt, auf dem anschließend die künstliche Zahnkrone gefertigt wird.

Grundsätzlich kommen unterschiedliche Materialien für die Herstellung einer künstlichen Zahnkrone in Frage. Metallbasierte Zahnkronen werden beispielsweise aus hochwertigen Goldlegierungen, einer goldreduzierten Legierung, Titan oder einer nicht Edelmetalllegierung hergestellt. Ebenso sind Vollkeramikzahnkronen bekannt, die üblicherweise einen Kern aufweisen, der mithilfe mechanischer Fertigungsverfahren bearbeitet wird und anschließend mit Keramik verblendet wird. Für derartige Zahnkronen besteht ebenfalls die Möglichkeit eine so genannte Presskeramik herzustellen, wobei bei hoher Temperatur aus flüssiger Keramik die Krone gepresst wird. In diesem Fall ist ein weiteres Verblenden nicht mehr unbedingt nötig, bietet jedoch den Vorteil, die optischen Eigenschaften der Sichtfläche der Zahnkrone an die entsprechenden natürlichen Eigenschaften eines Zahns anzupassen. Auf diese Weise kann eine natürliche Farbwirkung nachgebildet werden. Vollkeramikkronen haben vor allem den Vorteil, dass sie aufgrund ihrer im Vergleich zu metallischen Kronen geringeren Wärmeleitfähigkeit weniger thermische Reize verursachen.

In diesem Zusammenhang ist aus der DE 36 05 437 A1 ein Verfahren sowie ein keramischer Werkstoff zum Herstellen von keramischem Zahnersatz bekannt. Die beschriebene technische Lösung beruht auf einer speziellen Kombination unterschiedlicher dentalkeramischer Werkstoffe. Die verwendeten Werkstoffe besitzen eine hohe mechanische Festigkeit und der Wärmeausdehnungskoeffizient dieser Trägerwerkstoffe wurde konnte so weit angehoben werden, dass er sowohl mit üblichen aufbrennfähigen metallischen Dentalgusswerkstoffen als auch üblichen keramischen Verblendmassen kompatibel ist. Die grundlegende Idee der beschriebenen technischen Lösung besteht hierbei darin, dass bei der Herstellung von festsitzendem Zahnersatz die Kronenränder und mechanisch hochbeanspruchte Bereiche von Zwischengliedern aus Dentalgusswerkstoffen hergestellt werden, während die übrigen Bereiche vollkeramisch gefertigt werden. Hierdurch besteht die Möglichkeit, dass der metallische Kronenrand im Bereich von Brückenzwischengliedern entsprechend der höheren Belastung stärker dimensioniert wird. Der verwendete dentalkeramische Werkstoff zeichnet sich dadurch aus, dass er bei Brenntemperatur mit aufbrennfähigen Dentalgusswerkstoffen reagiert und einen auf diese Gusswerkstoffe abgestimmten Wärmeausdehnungskoeffizienten hat.

Des Weiteren ist aus der WO2012/115623A1 eine künstliche Zahnkrone mit einem verbesserten Befestigungselement bekannt. Die in dieser Druckschrift beschriebene Zahnkrone verfügt als Grundwerkstoff über Zirkon, das bevorzugt weitere Stabilisatoren aufweist. Wesentliches Merkmal der beschriebenen technischen Lösung ist, dass die künstlichen Zahnkronen im Bereich einer Ausnehmung, innerhalb der der Zahnstumpf des Patienten positioniert und befestigt wird, über ein speziell geformtes Befestigungselement, insbesondere in S-Form, verfügen. Das derart in die künstliche Zahnkrone integrierte Befestigungselement soll die Stabilität der am Zahnstumpf befestigten Zahnkrone erhöhen, da Relativbewegungen zwischen der Zahnkrone und dem Zahnstumpf erschwert werden.

Aus der WO 2013/127931 A1 ist ein Rohling sowie dessen Bearbeitung zur Herstellung von dentalen Formteilen bekannt. Der Rohling weist wenigstens einen Vorformling eines dentalen Formteils auf, aus dem durch mechanische Bearbeitung das benötigte Formtel hergestellt wird.

Auch aus der US 5,939,211 A ist ein Rohling, der zur Herstellung eines dentalen Formteils verwendet wird, bekannt. Durch ein subtraktives Herstellungsverfahren wird die gewünschte Kontur erzeugt wird, wobei sich der Rohling dadurch auszeichnet, dass dieser Bereiche mit unterschiedlichen Materialien aufweist. Hierbei verfügt der Rohling über einen Verstärkungsbereich, der von einem weiteren Material umgeben ist, das zur Herstellung des gewünschten Formteils bearbeitet wird.

Ferner ist aus der WO 2008/114142 A1 die Herstellung eines keramisches Grundgerüsts für ein Zahnersatzformteil bekannt. Gemäß der vorgeschlagenen technischen Lösung wird zunächst ein Grünkörper hergestellt, der zunächst bei vergleichsweise geringen Temperaturen um etwa 300 °C gesintert wird. Dieser vorgesinterte Grünkörper wird durch mechanische Bearbeitung in die gewünschte Form eines keramischen Grundgerüsts gebracht und dann, um die gewünschte Härte zu erzeugen, bei einer Temperatur von mindestens 900 °C gesintert. Demgegenüber hat metallischer Zahnersatz vor allem die beschriebenen Nachteile in Bezug auf die vergleichsweise hohe Leitfähigkeit von Wärme und elektrischem Strom.

Von den zuvor beschriebenen, für die Herstellung von Zahnersatz verwendeten Materialien weist keramischer Zahnersatz zwar Vorteile im Hinblick auf die bessere Ästhetik auf, ist aber in Bezug auf seine Festigkeit und die Kronengestaltung metallischem Zahnersatz unterlegen. Darüber hinaus sind regelmäßig die verwendeten Verblendmaterialien entweder zu hart oder zu elastisch und verfügen nicht über die physikalischen Eigenschaften der natürlichen Zahnsubstanz.

Demgegenüber hat metallischer Zahnersatz vor allem den Nachteil, über eine vergleichsweise hohe Wärmeleitfähigkeit sowie elektrische Leitfähigkeit zu verfügen. Ferner weisen metall- bzw. zirkongetragene Verblendkronen oder Verblendbrücken, die über keramische Verblendungen verfügen, ein vergleichsweise hohes Chipping-Risiko auf, neigen also zu Abplatzungen.

Weiterhin ist zu beachten, dass sich Zähne im Laufe eines Lebens im Wege von Abrasionserscheinungen abnutzen. Zahnersatzmaterialien sollten dieses natürliche Abrasionsverhalten imitieren. Dazu müssen sie einen dem natürlichen Zahnschmelz ähnlichen Härtegrad aufweisen. Insbesondere Zirkon weicht mit einem zehnmal höheren Härtegrad deutlich von dieser Empfehlung ab. Hieraus können Schäden an den verbliebenden Zähnen und / oder Fehlstellungen des Kiefers führen. In diesem Zusammenhang kann es sogar vorkommen, dass monolithische Zirkonoxid-Kronen (Bruxzir®-Krone) den Antagonisten beschädigen, so dass Kiefergelenkbeschwerden und Funktionsstörungen, die den gesamten Organismus, insbesondere bei Kindern, betreffen, eintreten können. Viele Patienten klagen darüber hinaus über Adaptionsschwierigkeiten.

Ausgehend von den aus dem Stand der Technik bekannten technischen Lösungen sowie den zuvor geschilderten Problemen liegt der Erfindung die Aufgabe zugrunde, ein besonders geeignetes Verfahren zur Herstellung einer Zahnkrone bzw. einer Abdeckung für einen Zahnstumpf, einen Stiftaufbau oder ein Zahnimplantat anzugeben, bei dem mit verhältnismäßig einfachen Mitteln ein hochgenaues Zahnersatzbauteil gefertigt wird. Hierbei soll gleichfalls ein Dentalwerkstoff zur Verfügung gestellt werden, der sich auf bevorzugte Weise bearbeiten lässt und gleichzeitig die Elastizität des Dentinkerns sowie die Härte und Abrasionsfestigkeit des Zahnschmelzes zumindest weitgehend imitiert.

Mithilfe des anzugebenden Verfahrens soll es möglich sein, eine Vielzahl von Standardrohlingen unterschiedlicher Größe bereitzustellen und auf einfache Weise derart zu bearbeiten, dass eine einfache, schmerzfreie und für den Patienten weiterhin nebenwirkungsarme Befestigung der Zahnkrone im Mund gewährleistet ist. Ein weiterer Aspekt der vorliegenden Erfindung liegt in diesem Zusammenhang darin, dass die im Mund befestigte Zahnkrone hier noch weiter an die Bedürfnisse des Patienten angepasst werden kann. Des Weiteren soll mit dem neuen Verfahren eine Zahnkrone herstellbar sein, die sich auf bevorzugte Weise auch für Kinderzähne eignet, insbesondere auch hier nicht zu einseitigem Versatz des Kondylus am Kiefergelenk und zu Fehlbelastungen führen und eine gleichfalls ästhetische und funktionelle Alternative zu den bekannten Zahnkronen darstellen. Für den Einsatz derart hergestellter Zahnkronen ist es weiterhin erforderlich, dass hiermit auch Zahnstümpfe von Kindern, die an der Molar-Incisor-Hypomineralisation (MIH) leiden, problemlos, insbesondere ohne Einsatz zusätzlicher Ätzverfahren, einsetzbar sind.

Die vorstehend geschilderte Aufgabe wird mit einem Verfahren gemäß Anspruch 1 gelöst. Ferner wird die der Erfindung zugrundeliegende Aufgabe mithilfe einer künstlichen Zahnkrone nach Anspruch 8 und einer Überdeckung gemäß Anspruch 7 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft ein Verfahren zur Herstellung einer künstlichen Zahnkrone mit einer Befestigungsfläche zur mittelbaren oder unmittelbaren Befestigung der Zahnkrone auf einem Zahnstumpf, einem Stiftaufbau oder einem Zahnimplantat. Es wird ein Kronengrundkörper, der wenigstens teilweise keramisches Material aufweist, bereitgestellt und mittels wenigstens eines formändernden Verfahrensschritts in-vitro verformt. Das erfindungsgemäße Verfahren zeichnet sich dadurch aus, dass der Kronengrundkörper wenigstens bereichsweise aus einem mit keramischem Material verstärkten Kompositkunststoff, der kein Bisphenol A enthält, hergestellt ist, der bezogen auf seine Gesamtmasse 75 bis 80% Kunststoff, 18 bis 22 % anorganische Füllstoffe und 1,5 bis 3% anorganische Pigmente aufweist, dass dieser Kronengrundkörper in-vitro mittels einer Spannvorrichtung eingespannt wird und durch subtraktives Zerspanen des Kronengrundkörpers eine benötigte Kronengeometrie erzeugt wird. Vorzugsweise erfolgt die zerspanende Bearbeitung des Kronengrundkörpers bzw. Fräsrohlings zumindest zeitweise mittels einer 5-Achs-CNC-Simultanfräse. Gemäß einer besonders geeigneten Ausführungsform der Erfindung erfolgt die Bearbeitung des Kronengrundkörpers zumindest zeitweise mit Hilfe eines kreuzverzahnten Fräskopfs, wobei insbesondere ein Titan-Fräskopf mit Härtegrad 4 geeignet ist.

Für die Herstellung entsprechender Zahnkronen aus einem Grundkörper mit keramisch verstärktem Kompositkunststoff eignen sich besonders Fräser mit einem Fräskopf, der einen Kopfdurchmesser von 0,6 mm bis 2,5 mm aufweist. Ganz besonders eignen sich Fräsköpfe mit einem Verhältnis des Kopf- zum Schaftdurchmesser mit 0,6 mm zu 6,0 mm, 1,0 mm zu 6,0 mm sowie 2,5 mm zu 6,0 mm.

Für die zusätzliche Bearbeitung des Kronengrundkörpers zur Herstellung einer Zahnkrone ist es weiterhin vorteilhaft, dass der Kronengrundkörper wenigstens teilweise geschliffen und / oder poliert wird.

Zur Realisierung des erfindungsgemäßen Herstellungsverfahrens wird ein biokeramisches Hochleistungskomposit zur Verfügung gestellt. Der Verbundwerkstoff stellt eine hochwertige Alternative zur Vollkeramik dar und ist aufgrund seiner hohen Abrasionsstabilität, Plaqueresistenz und Biokompatibilität auch für Langzeitprovisorien geeignet. Aus dem zuvor beschriebenen Material verwendete Fräsrohlinge sind besonders geeignet für die Bearbeitung mithilfe geeigneter CAD- sowie CAM-Techniken. Um eine möglichst naturnahe Optik der hergestellten Zahnkrone zu gewährleisten, werden bevorzugt Kronengrundkörper in den Vita-Farben A1 bis A4, B1 bis B4, C1 bis C4 und D1 bis D4 verwendet.

Gemäß einer weiteren besonderen Ausführungsform der Erfindung werden Zahnkronen für die ersten Kinderzähne, die so genannten Milchzähne 51, 52, 53, 51, 55, 61, 62, 63, 64, 65, 71, 72, 73, 74, 75, 81, 82, 83, 84 und 85 hergestellt. Besonders bevorzugt wird auch eine Zahnkrone für den 6-Jahresmolaren, also den ersten bleibenden Zahn, der sich im gleichen Zeitraum wie die Milchzähne im Mund befindet.

In einer weiteren Ausführungsform der Erfindung werden jeweils Kronensätze mit Kronen in sechs verschiedenen Längen gefertigt. Bevorzugt werden für jeden Kronensatz jeweils drei Kronen mit unterschiedlichem Umfang, die die gleiche Länge aufweisen, hergestellt. Ein Standardkronensatz enthält somit vorzugsweise 18 unterschiedliche künstliche Zahnkronen.

In einer speziellen Ausführungsform der Erfindung wird durch Fräsen, insbesondere durch 5-Achs-CNC-Simultanfräsen eine zervikale Wandstärke hergestellt, die mindestens 0,6 mm stark ist. Okklusal wird eine Mindestwandstärke gefräst, die wenigstens 1,0 mm beträgt. Die Mindestwandstärke am Kronenrand beträgt 0,2 mm. Die Verbindungsquerschnitte im Frontzahnbereich sind auf vorteilhafte Weise größer als 10 mm² und im Seitenzahnbereich größer als 15 mm².

Gemäß einer vorteilhaften Weiterbildung des erfindungsgemäßen Verfahrens wird mit geringen Umdrehungszahlen, minimaler Anpresskraft und ausreichend Kühlung gefräst. Vorzugsweise erfolgen eine Vorpolitur mit Polierbürsten und ein Hochglanzpolieren mit Polierschwabbel. Durch eine sorgfältig durchgeführte Politur kann insbesondere sichergestellt werden, dass eine Plaque-Akkumulation zumindest stark vermindert wird.

In einer ganz speziellen Ausführungsform wird der zerspanend bearbeitete Grundkörper mit wenigstens einem Verblendkomposit verblendet.

Auf bevorzugte Weise wird ein Kronengrundkörper, der wenigstens bereichsweise Polymethylmethacrylat (PMMA) aufweist, bereitgestellt. Weiterhin ist bzw. sind in den Grundkörper vorzugsweise wenigstens bereichsweise anorganisches Füllmaterial und / oder anorganische Pigmente eingebracht. Gemäß einer ganz speziellen Ausführungsform wird ein Grundkörper zerspanend in seine Endform überführt, der bezogen auf seine Gesamtmasse 78% eines keramisch verstärkten Kunststoffkomposits bzw. eines biokeramischen Hochleistungskomposits aufweist.

Im Folgenden sind einzelne Eigenschaften eines bevorzugt verwendeten biokeramischen Hochleistungskomposits tabellarisch aufgeführt:

| | |
|---|---|
| Elastizitäts-Modul | 3050 MPa |
| Biegefestigkeit β_{B} | 72 MPa |
| Vickers-Härte | 196 MPa |
| Restmonomer | < 1,0 % |
| Wasserlöslichkeit L | 1,6 µg / mm³ |

Auf bevorzugte Weise wird die künstliche Zahnkrone in Bezug auf den zu überdeckenden Zahnstumpf mit einer Spielpassung ausgeführt, um weitere Komponenten zwischen der Zahnkrone und dem Zahnstumpf vorsehen zu können. Diesbezüglich bezieht sich die Erfindung auch auf ein Verfahren zur Herstellung einer Überdeckung, die mittelbar oder unmittelbar an einem Zahnstumpf, einem Stiftaufbau oder an einem Zahnimplantat befestigbar ist, bei dem auf die Befestigungsfläche einer nach einem Verfahren gemäß der vorangegangenen Beschreibung hergestellten künstlichen Zahnkrone zumindest bereichsweise eine Kompositschicht, die ein Acrylatharz aufweist, aufgebracht wird. Bevorzugt wird auf die Befestigungsfläche der Zahnkrone, die sich innerhalb einer Ausnehmung der Zahnkrone zur Aufnahme eines Zahnstumpfs, eines Stiftaufbaus oder eines Zahnimplantats der Zahnkrone befindet, eine Kompositschicht aufgebracht, die ein lonomerglas in einer Bis-GMA-basierten Matrix aus Dentalharzen, Aktivatoren und Additiven aufweist. Mithilfe einer entsprechend Kompositschicht kann die zerspanend hergestellte Spielpassung der Zahnkrone an die Form des zu überdeckenden Zahnstumpfes, Stiftaufbaus oder Zahnimplantats angenähert werden.

Durch das Vorsehen einer Überdeckung, die aus einer Zahnkrone und der zuvor beschriebenen dualhärtenden Kompositschicht besteht, kann schließlich ein Zahnstumpf, Stiftaufbau oder Zahnimplantat im Mund des Patienten überdeckt werden, ohne dass weitere Ätz- oder Bondingschritte erforderlich sind. Nach dem Einsetzen einer erfindungsgemäß hergestellten Zahnkrone und der Aushärtung der Kompositschicht besteht die Möglichkeit, die Wandungen der Krone soweit auszudünnen, dass die Standkraft durch das Komposit getragen wird. Auch der nachträgliche Aufbau im Mund ist problemlos möglich. Das gilt nicht nur für Milchzähne, sondern sowohl für die Sechs-Jahres-Molaren als auch für die bleibenden Eckzähne. Aufgrund der erfindungsgemäßen Herstellung einer Überdeckung, bestehend aus Zahnkrone, deren Ausnehmung mit Spielpassung ausgeführt ist, und einer speziellen Kompositschicht, ist eine besondere Anpassung der Überdeckung an die Außenkontur eines Zahnstumpfes möglich. Insbesondere besteht im Weiteren die Möglichkeit, die Wandungen der Zahnkrone soweit auszudünnen, dass die Wandstandkraft durch die dualhärtende Kompositschicht getragen wird. Die Kronenpassung kann somit individualisiert hergestellt werden, obgleich die Grundform der Zahnkrone standartisiert ist. Die Gefahr der Bildung von Sekundärkaries, wie sie unter den bislang verwendeten Zahnkronen auftreten kann, wird hierdurch minimiert. Durch die Herstellung einer Zahnkrone, deren Ausnehmung mit Spielpassung in bezug auf den zu überkronenden Zahnstumpf ausgeführt ist, und die Verwendung einer Kompositschicht zur Größenanpassung wird es trotz Herstellung von standardisierten Zahnkronen möglich, individuelle Überdeckungen für Zähne, insbesondere Kinderzähne bereitzustellen.

Die nach dem erfindungsgemäßen Verfahren hergestellte Zahnkrone sowie eine Überdeckung, die eine erfindungsgemäße Zahnkrone sowie die oben angegebene Kompositschicht aufweist, sind besonders geeignet, um Kinderzähne, die unter Molar-Incisor-Hypomineralisation (MIH) leiden, zu überkronen. Die mit dieser Krankheit befallenen Zähne sind nicht ausreichend mineralisiert, sehen käsig-trüb aus und können beim bloßen Kauen zerbröckeln.

Da die bislang für Kinder verwendeten künstlichen Zahnkronen, die vielfach Kunststoffe mit der Chemikalie Bisphenol A enthalten, die im Verdacht steht, die Mineralisation von Zähnen zu stören, eignen sich die bislang bekannten Zahnkronen nicht für Kinder. Darüber hinaus sind Folgeerkrankungen bei Kindern aufgrund des Einsatzes entsprechender Kunststoffe nicht ausgeschlossen. Molarenkronen aus edelmetallfreien Materialien (NEM) oder Zirkon, wie sie momentan auf dem Markt erhältlich sind, können bei Kindern zu Kiefergelenksproblemen führen. Dies wird mit einer auf erfindungsgemäße Weise hergestellten Zahnkrone vermieden.

Im Folgenden wird die Erfindung ohne Beschränkungen des allgemeinen Erfindungsgedankens anhand eines Ausführungsbeispiels unter Bezugnahme auf die Figuren näher erläutert. Dabei zeigt:
- Fig. 1:: Blockschaltbild zur Darstellung des Ablaufs des erfindungsgemäßen Verfahrens zur Herstellung einer künstlichen Zahnkrone
- Fig. 2:: Draufsicht eines Rohlings mit gefrästen Zahnkronen, die Standardmaße aufweisen, für Kinder sowie
- Fig. 3:: Ansicht auf die Kaufläche und die Unterseite einer Überdeckung, die eine künstliche Zahnkrone sowie eine Kompositschicht aufweist.

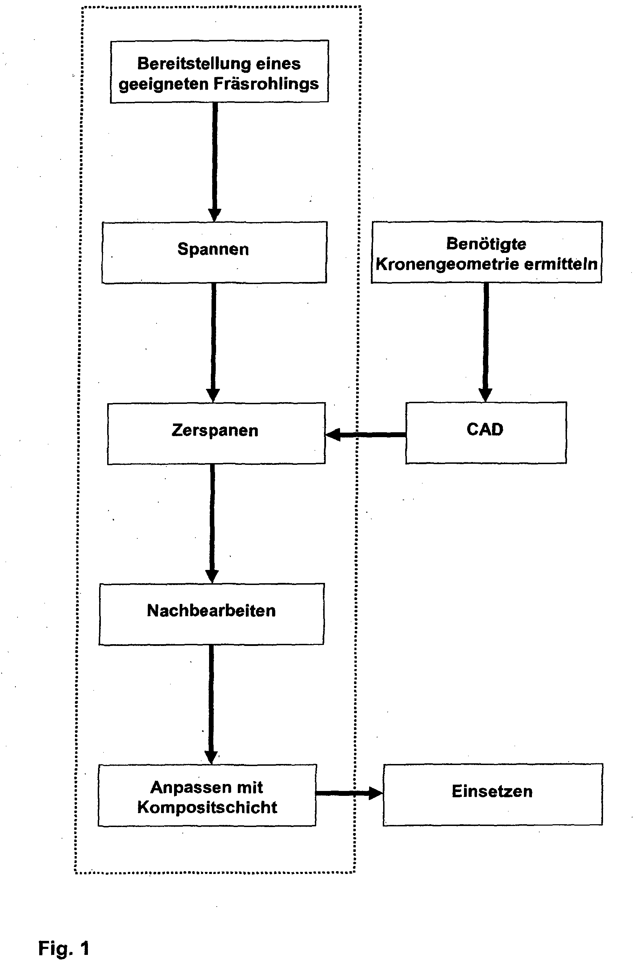

Figur 1 zeigt in einem Blockschaltbild die einzelnen Verfahrensschritte und deren Abfolge bei der Herstellung einer künstlichen Zahnkrone sowie einer Überdeckung, die eine derartige Zahnkrone enthält. Wesentlicher Bestandteil des beschriebenen Herstellungsverfahrens ist die Bereitstellung eines Halbzeugs bzw. Rohlings aus einem speziellen Material, nämlich einen keramisch verstärkten Kunststoffkomposit, das letztendlich eine besonders geeignete Bearbeitung und gleichzeitig die Realisierung eines für den Patienten besonders vorteilhaften Zahnersatzes ermöglicht.

Zunächst wird mittels eines scheibenförmigen Rohlings eine Vielzahl von Kronengrundkörpern 2 bereitgestellt. So ist es in Abhängigkeit der Größe der einzelnen Zahnkronen 1 und des bereitgestellten Rohlings 8 möglich, aus einem Rohling 8 eine Vielzahl von künslichen Zahnkronen 1, eventuell sogar einen Kronensatz herzustellen. Ein Kronensatz enthält Zahnkronen 1 in sechs verschiedenen Längen, wobei jeweils drei Zahnkronen 1 mit unterschiedlichem Umfang und gleicher Länge vorgesehen sind, so dass ein derart standardisierter Zahnkronensatz über insgesamt achtzehn Zahnkronen 1 verfügt.

Der Rohling 8 und damit die Grundkörper 2 der verschiedenen zu fertigenden Zahnkronen 1 sind aus einem keramisch verstärkten Kompositkunststoff hergestellt. Die Kronengrundkörper 2 enthalten ein Polymethylmethacrylat (PMMA) aufweist, bereitgestellt. Ferner sind in den Grundkörper anorganisches Füllmaterial und anorganische Pigmente eingebracht. bezogen auf die Gesamtmasse eines Kronengrundkörpers 2 enthält dieser in dem dargestellten Ausführungsbeispiel 78% Polymethylmethacrylat (PMMA), 20 % anorganische Füllstoffe und 2% anorganische Pigmente. Damit die künstlichen Zahnkronen 1 auch optisch zumindest weitgehend einem natürlichen Zahn entsprechen, werden Kronengrundkörper 2 in den Vita-Farben A1 bis A4, B1 bis B4, C1 bis C4 und D1 bis D4 für die Herstellung künstlicher Zahnkronen verwendet.

In einem nächsten Schritt wird der Rohling 8 eingespannt und die einzelnen Kronengrundkörper 2 derart durch 5-Achs-CNC-Simultanfräsen subtraktiv zerspant, dass schließlich die Zahnkronen 1 der gewünschten Geometrie aus dem Rohling 8 herausgearbeitet sind. Die Zahnkronen 1 sind dann nur noch über kleine Stege 3, die sich in dem zerspanten Bereich 4 befinden, an dem Rohling 8 befestigt. Die Zahnkronen 1 werden mit einer zervikalen Wandstärke von mindestens 0,6 mm gefertigt. Okklusal wird eine Mindestwandstärke gefräst, die wenigstens 1,0 mm beträgt. Die Mindestwandstärke am Kronenrand beträgt 0,2 mm. Die Verbindungsquerschnitte im Frontzahnbereich sind auf vorteilhafte Weise größer als 10 mm² und im Seitenzahnbereich größer als 15 mm².

Das Zerspanen der Grundkörper 2 erfolgt durch Fräsen mit geringen Umdrehungszahlen, minimaler Anpresskraft und ausreichend Kühlung. Nachdem die Stege 3 getrennt und eine Zahnkrone 1 dem Rohling entnommen wurde, erfolgen eine Vorpolitur mit Polierbürsten und ein Hochglanzpolieren mit Polierschwabbel. Durch diese Politur kann insbesondere sichergestellt werden, dass eine Plaque-Akkumulation zumindest stark vermindert wird.

In Bezug auf den zu überdeckenden Zahnstumpf, Stiftaufbau oder Zahnimplantat wird die künstliche Zahnkrone mit einer Spielpassung ausgeführt, um weitere Komponenten zwischen der Zahnkrone und dem Zahnstumpf vorsehen zu können.

In einem weiteren Verfahrensschritt wird in Abhängigkeit des Außendurchmessers des zu überdeckenden Zahnstumpfs, Stiftaufbaus oder Zahnimplantats zumindest bereichsweise eine Kompositschicht 5, die ein Acrylatharz aufweist, auf die künstliche Zahnkrone 1 aufgebracht wird. Die Kompositschicht 5 wird auf die Befestigungsfläche der Zahnkrone 1, die sich innerhalb einer Ausnehmung 6 der Zahnkrone 1 zur Aufnahme eines Zahnstumpfs, eines Stiftaufbaus oder eines Zahnimplantats der Zahnkrone befindet, aufgebracht. Es wird eine Kompositschicht verwendet, die ein lonomerglas in einer Bis-GMA-basierten Matrix aus Dentalharzen, Aktivatoren und Additiven aufweist. Mithilfe einer entsprechenden Kompositschicht kann die zerspanend hergestellte Spielpassung der Zahnkrone an die Form des zu überdeckenden Zahnstumpfes, Stiftaufbaus oder Zahnimplantats angenähert werden.

Der zerspanenden Bearbeitung des Kronengrundkörpers 2 wird eine Fertigungszeichnung zugrunde gelegt, die mittels CAD (Computer-Aided Design) erstellt wurde. Den im CAD-System verwendeten Ausgangsdaten liegen die ermittelte Größe bzw. Kontur des zu überdeckenden Zahnstumpfs, Stiftaufbaus oder Zahnimplantats zugrunde. Die Datenverarbeitung erfolgt digital, wobei entsprechend generierte Steuersignale drahtgebunden oder drahtlos an die Steuereinheit der CNC-Fräsmaschine übertragen werden.

Figur zeigt einen Rohling 8, aus dem bereits eine Vielzahl von Zahnkronen 1 herausgefräst worden sind. Diese Zahnkronen 1 werden lediglich noch mittels geeigneter Stege 3 im Rohling 8 gehalten. Das übrige Material in dem Bereich 4 zwischen Rohling 8 und fertig gestellter Zahnkrone 1 wurde zerspant und zumindest teilweise mit der Bohrflüssigkeit abgeführt.

Punktiert ist ein noch nicht bearbeiteter Grundkörper 2 dargestellt. In diesem Bereich wird zu einem späteren Zeitpunkt eine künstliche Zahnkrone 1 aus dem Rohling 8 herausgefräst.

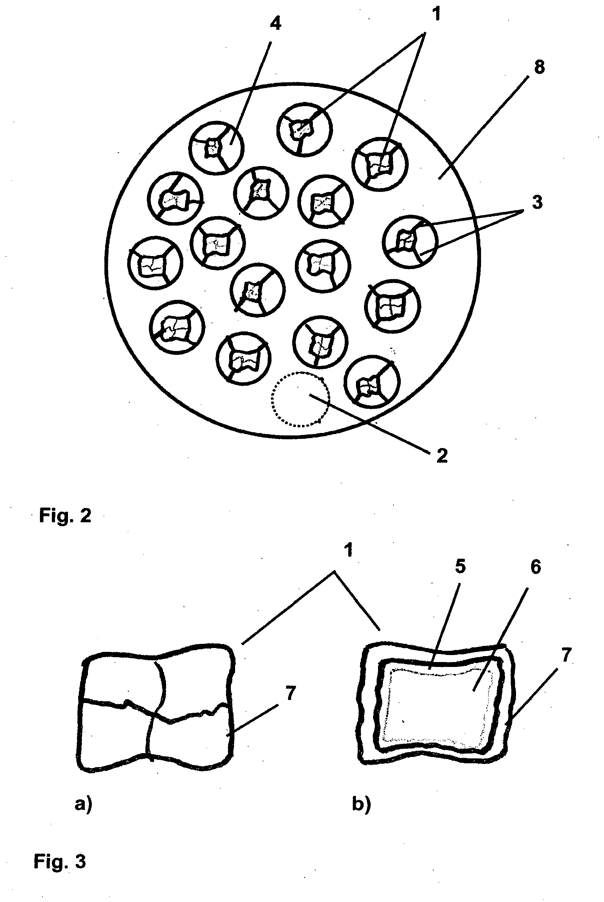

Weiterhin ist in Figur 3 eine einzelne Überdeckung für einen Zahnstumpf, Stiftaufbau oder ein Zahnimplantat dargestellt, die eine Zahnkrone 1 und eine daran befestigte Kompositschicht 5 aufweist Die Kompositschicht 5 verfügt über ein lonomerglas in einer Bis-GMA-basierten Matrix aus Dentalharzen, Aktivatoren und Additiven.

Figur 3 a) zeigt eine Draufsicht auf die Kaufläche der künstlichen Zahnkrone während in Figur 3 b) die Unterseite der Zahnkrone 1 mit der dort befindlichen Ausnehmung 6 zur Aufnahme eines Zahnstumpfs, Stiftaufbaus oder eines Zahnimplantats. Mithilfe einer entsprechenden Kompositschicht kann die zerspanend hergestellte Spielpassung der Zahnkrone an die Form des zu überdeckenden Zahnstumpfes, Stiftaufbaus oder Zahnimplantats angenähert werden.

Viele der bekannten Zahnkronen eignen sich nicht für Kinder, da sie Kunststoffe mit der Chemikalie Bisphenol A enthalten, die im Verdacht steht, die Mineralisation von Zähnen zu stören. Auch Folgeerkrankungen sind bei Kindern aufgrund des Einsatzes entsprechender Kunststoffe nicht ausgeschlossen. Darüber hinaus können Molarenkronen aus edelmetallfreien Materialien (NEM) oder Zirkon, wie sie momentan auf dem Markt erhältlich sind, bei Kindern zu Kiefergelenksproblemen führen, die sich insbesondere in der Wachstumsphase sehr negativ auf andere Gelenke auswirken können. Diese Probleme werden wird mit einer auf erfindungsgemäße Weise hergestellten Zahnkrone vermieden.

### Bezugszeichenliste

- 1: künstliche Zahnkrone
- 2: Kronengrundkörper
- 3: Steg
- 4: Zerspanter Bereich
- 5: Kompositschicht
- 6: Ausnehmung in der Zahnkrone
- 7: Randbereich der Zahnkrone
- 8: Rohling

## Patentansprüche

1. Verfahren zur Herstellung einer künstlichen Zahnkrone (1) mit einer Befestigungsfläche zur mittelbaren oder unmittelbaren Befestigung der Zahnkrone (1) auf einem Zahnstumpf, einem Stiftaufbau oder einem Zahnimplantat, bei dem ein Kronengrundkörper (2), der wenigstens teilweise keramisches Material aufweist, bereitgestellt wird und mittels wenigstens eines formändernden Verfahrensschritts in-vitro verformt wird, **dadurch gekennzeichnet, dass** der verwendete Kronengrundkörper (2) wenigstens bereichsweise aus einem mit keramischem Material verstärkten Kompositkunststoff, der kein Bisphenol A enthält, hergestellt ist, der bezogen auf seine Gesamtmasse 75 bis 80% Kunststoff, 18 bis 22 % anorganische Füllstoffe und 1,5 bis 3% anorganische Pigmente aufweist, dass der Kronengrundkörper (2) in-vitro mittels einer Spannvorrichtung eingespannt wird und dass durch subtraktives Zerspanen des Kronengrundkörpers (2) eine benötigte Kronengeometrie erzeugt wird.

2. Verfahren nach Anspruch 1,
**dadurch gekennzeichnet, dass** der Kronengrundkörper (2) wenigstens zeitweise mit einem kreuzverzahnten Fräskopf, insbesondere einem Titan-Fräskopf mit Härtegrad 4, zerspant wird.

3. Verfahren nach Anspruch 1 oder 2,
**dadurch gekennzeichnet, dass** der Kronengrundkörper (2) wenigstens zeitweise mit einem Fräskopf, der einen Kopfdurchmesser von 0,6 bis 2,5 mm aufweist, zerspant wird.

4. Verfahren nach einem der Ansprüche 1 bis 3,
**dadurch gekennzeichnet, dass** der Kronengrundkörper (2) wenigstens teilweise geschliffen und/oder poliert wird.

5. Verfahren nach einem der Ansprüche 1 bis 4,
**dadurch gekennzeichnet, dass** in den Kronengrundkörper (2) wenigstens bereichsweise Polymethylmethacrylat (PMMA) eingebracht wird.

6. Verfahren nach einem der Ansprüche 1 bis 5,
**dadurch gekennzeichnet, dass** in den Kronengrundkörper (2) wenigstens bereichsweise anorganisches Füllmaterial und/oder anorganische Pigmente eingebracht werden.

7. Überdeckung, die mittelbar oder unmittelbar an einem Zahnstumpf, einem Stiftaufbau oder an einem Zahnimplantat befestigbar ist, mit einer Zahnkrone, die mit einem Verfahren gemäß zumindest einem der Ansprüche 1 bis 6 hergestellt ist, und mit einer zumindest bereichsweise auf die Zahnkrone aufgebrachten Kompositschicht (5), die ein lonomerglas in einer Bis-GMA-basierten Matrix aus Dentalharzen, Aktivatoren und Additiven aufweist.

8. Zahnkrone aufweisend einen Grundkörper, der über einen mit keramischem Material verstärkten Kompositkunststoff, der kein Bisphenol A enthält und der bezogen auf seine Gesamtmasse 75 bis 80% Kunststoff, 18 bis 22 % anorganische Füllstoffe und 1,5 bis 3% anorganische Pigmente aufweist, verfügt, die zur Behandlung eines an Molar-Incisor-Hypomineralisation (MIH) erkrankten Kinderzahns geeignet ist.

## Claims

1. A method for producing an artificial tooth crown (1) having a mounting surface for direct or indirect attachment of the tooth crown (1) on a tooth stump, a pin structure, or a dental implant, wherein a crown base body (2), which at least partially comprises ceramic material, is provided and is deformed in vitro by means of at least one shape-altering procedural step,
**characterized in that** the crown base body (2) used is at least in certain areas made of a composite material reinforced with ceramic material, which does not contain bisphenol A, which, relative to its total mass, comprises 75 to 80 % of plastic, 18 to 22 % of inorganic fillers, and 1.5 to 3 % of inorganic pigments, that the crown base body (2) is clamped in vitro by means of a clamping device, and that a required crown geometry is generated by subtractive machining of the crown base body (2).

2. The method according to claim 1,
**characterized in that** the crown base body (2) is at least temporarily machined with a cross-cut milling head, in particular a titanium milling head with a hardness of 4.

3. The method according to claim 1 or 2,
**characterized in that** the crown base body (2) is at least temporarily machined with a milling head having a head diameter of 0.6 to 2.5 mm.

4. The method according to any one of claims 1 to 3,
**characterized in that** the crown base body (2) is at least partially ground and/or polished.

5. The method according to any one of claims 1 to 4,
**characterized in that**, at least in certain areas, polymethylmethacrylate (PMMA) is introduced into the crown base body (2).

6. The method according to any one of claims 1 to 5,
**characterized in that**, at least in certain areas, inorganic filling material and/or inorganic pigments are introduced into the crown base body (2).

7. An overlay, which is directly or indirectly attachable to a tooth stump, a pin structure, or a dental implant, with a tooth crown, which is produced using a method according to at least one of claims 1 to 6, and with a composite layer (5) applied onto the tooth crown at least in certain areas, comprising an ionomer glass in a Bis-GMA-based matrix made of dental resins, activators, and additives.

8. A tooth crown having a base body, which comprises a composite plastic reinforced with ceramic material, which does not contain bisphenol A and which, relative to its total mass, includes 75 to 80 % of plastic, 18 to 22 % of inorganic fillers, and 1.5 to 3 % of inorganic pigments, suitable for the treatment of a children's tooth diseased with molar incisor hypomineralization (MIH).

## Revendications

1. Procédé de fabrication d'une couronne dentaire artificielle (1) avec une surface de fixation pour la fixation directe ou indirecte de la couronne dentaire (1) sur un moignon de dent, sur un pivot ou sur un implant dentaire, dans le cadre duquel un corps de base de couronne (2) en matériau au moins partiellement céramique est prévu et est déformé in vitro au moyen d'au moins une étape de procédé destinée à changer la forme,
**caractérisé en ce que** ledit corps de base de couronne (2) utilisé est fabriqué au moins par zones à partir d'une matière plastique composite renforcée par un matériau céramique, laquelle ne contient pas de bisphénol A et présente, par rapport à sa masse totale, 75 à 80% de matière plastique, 18 à 22% de charges inorganiques et 1,5 à 3% de pigments inorganiques, **en ce que** ledit corps de base de couronne (2) est serré in vitro au moyen d'un dispositif de serrage et **en ce qu'**une géométrie de couronne requise est réalisée par voie d'usinage soustractif dudit corps de base de couronne (2).

2. Procédé selon la revendication 1,
**caractérisé en ce que** ledit corps de base de la couronne (2) est au moins temporairement usiné au moyen d'une fraise à dents croisées, en particulier une fraise en titane d'un degré de dureté 4.

3. Procédé selon la revendication 1 ou 2,
**caractérisé en ce que** ledit corps de base de la couronne (2) est au moins temporairement usiné au moyen d'une fraise ayant un diamètre de 0,6 à 2,5 mm.

4. Procédé selon l'une des revendications 1 à 3,
**caractérisé en ce que** ledit corps de base de la couronne (2) est au moins partiellement poncé et/ou poli.

5. Procédé selon l'une des revendications 1 à 4,
**caractérisé en ce que** du polyméthacrylate de méthyle (PMMA) est incorporé dans ledit corps de base de la couronne (2) au moins par endroits.

6. Procédé selon l'une des revendications 1 à 5,
**caractérisé en ce que** des charges inorganiques et/ou des pigments inorganiques sont incorporés dans ledit corps de base de la couronne (2) au moins par endroits.

7. Revêtement pouvant être fixé directement ou indirectement sur un moignon de dent, sur un pivot ou sur un implant dentaire, avec une couronne dentaire qui est fabriquée selon un procédé selon au moins l'une des revendications 1 à 6, et avec une couche composite (5) qui est appliquée au moins par zones sur ladite couronne dentaire et qui présente un verre ionomère dans une matrice à base de Bis-GMA composée de résines dentaires, d'activateurs et d'additifs.

8. Couronne dentaire comprenant un corps de base qui a une matière plastique composite renforcée par un matériau céramique, laquelle ne contient pas de bisphénol A et comprend, par rapport à sa masse totale, 75 à 80% de matière plastique, 18 à 22% de charges inorganiques et 1,5 à 3% de pigments inorganiques, couronne qui convient pour le traitement d'une dent d'enfant souffrant d'hypominéralisation des molaires et incisives (HMI).
